(19) Europäisches Patentamt
European Patent Office
Office européen des brevets

(11) **EP 2 195 758 B1**

(12) **EUROPÄISCHE PATENTSCHRIFT**

(45) Veröffentlichungstag und Bekanntmachung des Hinweises auf die Patenterteilung:
**01.05.2019** Patentblatt 2019/18

(21) Anmeldenummer: **08804949.9**

(22) Anmeldetag: **01.10.2008**

(51) Int Cl.:
**G16H 20/17** *(2018.01)* **A61M 5/168** *(2006.01)*

(86) Internationale Anmeldenummer:
**PCT/EP2008/063125**

(87) Internationale Veröffentlichungsnummer:
**WO 2009/047181 (16.04.2009 Gazette 2009/16)**

(54) **FÖRDERVORRICHTUNG UND VERFAHREN FÜR DIE ZUFÜHRUNG EINES MEDIKAMENTEN-LÖSUNGSGEMISCHES**

CONVEYOR APPARATUS AND METHOD FOR SUPPLYING A MEDICINE-SOLUTION MIXTURE

SYSTÈME DE TRANSPORT ET PROCÉDÉ POUR L'ADMINISTRATION D'UN MÉLANGE DE SOLUTIONS MÉDICAMENTEUSES

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **02.10.2007 DE 102007047353**

(43) Veröffentlichungstag der Anmeldung:
**16.06.2010** Patentblatt 2010/24

(73) Patentinhaber: **B. Braun Melsungen AG**
**34212 Melsungen (DE)**

(72) Erfinder:
• **LAUER, Hans-Martin**
**80997 München (DE)**
• **WUFKA, Matthias**
**80636 München (DE)**
• **HÖRNIG, Sebastian**
**85258 Weichs (DE)**
• **RÖTHLEIN, Doris**
**34121 Kassel (DE)**

(74) Vertreter: **Winter, Brandl, Fürniss, Hübner, Röss, Kaiser, Polte - Partnerschaft mbB**
**Patent- und Rechtsanwaltskanzlei**
**Alois-Steinecker-Strasse 22**
**85354 Freising (DE)**

(56) Entgegenhaltungen:
**WO-A-2004/069095     US-A1- 2003 032 867**
**US-B2- 6 827 702**

• **Howard Madsen ET AL: "The Hitchhiker's Guide to Parenteral Nutrition Management for Adult Patients", Nutritional Issues in Gastroenterology, Series No. 40, 31 July 2006 (2006-07-31), pages 46-68, XP055112697, Retrieved from the Internet: URL:http://www.medicine.virginia.edu/clini cal/departments/medicine/divisions/digesti ve-health/nutrition-support-team/nutrition -articles/MadsenArticle.pdf [retrieved on 2014-04-08]**
• **Gordon Sacks: "Drug-Nutrient Considerations in Patients Receiving Parenteral and Enteral Nutrition", NUTRITION ISSUES IN GASTROENTEROLOGY, Series No. 19, 31 July 2004 (2004-07-31), pages 39-48, XP055112707, Retrieved from the Internet: URL:http://www.medicine.virginia.edu/clini cal/departments/medicine/divisions/digesti ve-health/nutrition-support-team/nutrition -articles/SaksArticle.pdf [retrieved on 2014-04-08]**

**Beschreibung**

Die Erfindung betrifft eine Fördereinrichtung und ein Verfahren für die Zuführung eines Medikamenten-Lösungsgemisches aus einer Mehrzahl an Medikamenten-Wirkstofflösungen und weiterer Lösungsbestandteile in einen Körper mittels einer Mehrzahl an Zuführeinrichtungen, wobei die Medikamenten-Wirkstofflösungen verschiedene Medikamenten-Wirkstoffparameter und Inhaltsstoffe und die Lösungsbestandteile verschiedene Parameter und Inhaltsstoffe aufweisen, gemäß den Oberbegriffen der Patentansprüche 1 und 5.

Fördervorrichtungen für die Zuführung eines Medikamenten-Lösungsgemisches in einen Körper sind mehrfach bekannt.

US 2003/0032867 A1 offenbart beispielsweise ein Diabetes-Managementsystem und ein Verfahren zum Einstellen des Blutzuckerwerts eines Patienten. Dabei umfasst das System eine tragbare elektronische Vorrichtung und ein Datenbanksystem. Der Patient kann verschiedene Daten eingeben, auf Basis derer dann die benötigte Insulinmenge berechnet wird und mit Hilfe einer Insulinpumpe dem Patienten zugeführt werden kann. Das Datenbanksystem speichert sämtliche eingegebenen Daten und generiert einen Zeitstempel. Die Daten können auf einen Rechner geladen und dort analysiert werden.

US 6 827 702 B2 offenbart ein System und Verfahren zum Bereitstellen von Sicherheitsgrenzen zur Zuführung einer Infusion durch eine Infusionspumpe als Reaktion auf einen erfassten beispielsweise biologischen Zustand. Auf Basis der erhaltenen Daten wird eine Insulin-Zuführrate angepasst. Grundlage für die Berechnung sind personenspezifische Daten, die zu Beginn einprogrammiert werden.

WO 2004/069095 A offenbart ein System und ein Verfahren für eine Remote-Mehrzweck-Benutzeroberfläche für medizinische Geräte und Systeme mit medizinischen Geräten und/oder Medikamenten-Informationssystemen. In einer Datenbank werden Informationen über die Infusionspumpen, über Medikamente, Inhaltsstoffe, Konzentrationen und Wirkstoffe gespeichert. Bei den medizinischen Geräten kann es sich beispielsweise um Geräte zur parenteralen Ernährung handeln.

In Madsen, H. et al, "The Hitchhiker's Guide to Parenteral Nutrition Management for Adult Patients", Practical Gastroenterology, Juli 2006 ist beschrieben bei einer parenteralen Ernährung manche Medikamente beispielsweise Einfluss auf den Blutglucosegehalt haben.

In Sacks, G. S., "Drug-Nutrient Considerations in Patients Receiving Parenteral and Enteral Nutrition", Practical Gastroenterology, Juli 2004) ist beschrieben, was bei der Arzneimittelvergabe an Patienten, die parenteral oder enteral ernährt werden, beachtet werden muss.

Beispielsweise sind Vorrichtungen mit einer Mehrzahl an Infusions- und/oder Spritzenpumpen, wovon jede eine Lösung mit mindestens einem spezifischen Medikamentenwirkstoff einem Körper zuführt und hierdurch ein Medikamenten-Lösungsgemisch entsteht, bekannt. Zusätzlich werden weitere Lösungsbestandteile als Zusatzstoffe, wie Trägerlösungen und Konservierungsmittel, beigemischt, um hierdurch eine Konservierung des Medikamenten-Lösungsgemisches und Trägerstoffe für den Transport der Medikamentenwirkstoffe zu erhalten.

Derartige Infusionspumpen-Systeme werden häufig bei Patienten, die einer intensivmedizinischen Behandlung bedürfen, verwendet. Hierbei weisen die Infusionspumpen die Eigenschaften der kontinuierlichen und genauen Dosierung der Medikation in ihrer Zuführung auf. Um eine optimierte Abstimmung dieser Pumpen in ihrer Dosierung zu erreichen, werden die Pumpen in einem gemeinsamen Ordnungssystem integriert, welches üblicherweise eine zentrale Steuereinheit, eine Bedienungseinheit und eine Alarmierungseinheit aufweist. Hierdurch wurde es ermöglicht, dass sogar eine zweistellige Anzahl an verschiedenen Medikamenten einem Körper aufeinander abgestimmt und genau dosiert zugeführt werden kann.

Solche Pumpen weisen eine Förderrate, die als Volumen pro Zeiteinheit (1 ml/h) dargestellt ist auf. Demgegenüber wird in der Medizin für eine zugeführte Medikamentenlösung die Dosiseinheit, beispielsweise in mg/kgKG/24 h oder mmol/min verwendet. Demzufolge ist es erforderlich, die Dosiseinheit in eine Förderrate der Pumpe umzurechnen, was Aufgabe des behandelnden Arztes ist. Hierbei gilt folgende Formel:

$$Rate = \frac{Dosis}{Konzentration\_des\_Medikaments}$$

Bei einer derartigen Umrechnung treten häufig Rechenfehler auf. Mittlerweile werden demzufolge Infusionspumpen angeboten, die die Eingabe einer Dosiseinheit erlauben und automatisch die Umrechnung in eine Förderrate durchführen. Hierfür sind einige zusätzliche technische Merkmale notwendig, wie z. B. das Einbinden eines Barcodescanners, um einen von der Apotheke, aus welcher ein in der Pumpe einzulegender Behälter mit dem Medikament bezogen wird, angebrachten Barcode zu scannen. Dieser Barcode enthält Angaben über die Konzentration des Wirkstoffes des in der Spritze enthaltenen Medikamentes und über die Art des Medikamentes.

Nach erfolgtem Einscannen kann der Arzt die gewünschte Dosis für das ausgewählte Medikament in die Infusionspumpe mittels einer Eingabeeinrichtung eingeben, woraufhin die Pumpe mit der Umrechnung der Dosiseinheit in die Förderrate und das Verabreichen des Medikamentes beginnt.

Sowohl bei der Angabe der Konzentration des Wirkstoffes als auch bei der Eingabe der Dosiseinheit und der Umrechnung derjenigen in die Förderrate werden bisher ausschließlich die Hauptwirkstoffe des Medikamentes und damit des Medikamenten-Lösungsgemisches berücksichtigt und betrachtet. Dies ist häufig ausreichend, sofern lediglich oder vorrangig ein spezifisches Medikament verabreicht werden soll.

Hinzu kommt, dass bei der Verabreichung von Medikamenten mittels Pumpen häufig eine Bilanzierung zwischen den dem Patienten zugeführten Flüssigkeitsvolumina und den von den Patienten ausgeschiedenen Flüssigkeitsvolumina durchgeführt wird. Eine derartige Bilanzierung ermöglicht den Erhalt von Ergebnissen zu den Nierenfunktionen, verschiedenen Volumenverhältnissen und die Verwertung der infundierten Stoffe innerhalb des Körpers. Derartige von Pumpen selbsttätig durchgeführte Bilanzierungsrechnungen beruhen darauf, dass die infundierten Volumina bestimmt werden und ausschließlich der Hauptwirkstoff aufsummiert wird.

Demzufolge liegt der vorliegenden Erfindung die Aufgabe zugrunde, eine Fördervorrichtung und ein Verfahren für die Zuführung eines Medikamenten-Lösungsgemisches aus einer Mehrzahl an Medikamenten-Wirkstofflösungen und weiterer Lösungsbestandteile zur Verfügung zu stellen, die/das insbesondere im Bereich der künstlichen Ernährung eine umfassendere Berücksichtigung der einzelnen Bestandteile eines Medikamenten-Lösungsgemisches für nachfolgende Berechnungen von Parametern beziehungsweise Werten ermöglicht.

Diese Aufgabe wird vorrichtungsseitig auf die Merkmale des Patentanspruches 1 und verfahrensseitig durch die Merkmale des Patentanspruches 5 gelöst.

Ein wesentlicher Punkt liegt darin, dass bei einer Fördereinrichtung für die Zuführung eines Medikamenten-Lösungsgemisches aus einer Mehrzahl an Medikamenten-Wirkstofflösungen, die verschiedene Medikamenten-Wirkstoffparameter und Inhaltsstoffe aufweisen, und weiterer Lösungsbestandteile, die verschiedene Parameter und Inhaltsstoffe aufweisen, in einen Körper mittels einer Mehrzahl an Zuführeinrichtungen von mindestens zwei, vorzugsweise sämtlichen, Medikamenten-Wirkstofflösungen und Lösungsbestandteilen die Parameter und/oder die Inhaltsstoffe in mindestens einer Speichereinrichtung gespeichert sind, eine Auswahleinrichtung zum Auswählen mindestens eines spezifischen Parameters und/oder Inhaltsstoffes von jeder Medikamenten-Wirkstofflösung und/oder den Lösungsbestandteilen mit der Speichereinrichtung verbunden ist und eine Berechnungseinrichtung zum Berechnen mindestens eines weiteren Parameters aus sämtlichen ausgewählten Parametern für die Steuerung des Betriebs mindestens einer weiteren Zuführeinrichtung zum Zuführen mindestens eines weiteren Medikamenten-Lösungsgemisches zu dem Körper mit der Auswahleinrichtung verbunden ist.

Konkret ist eine Fördervorrichtung für die Zuführung eines Medikamenten-Lösungsgemisches aus einer Mehrzahl an Medikamenten-Wirkstofflösungen und weiteren Lösungsbestandteilen vorgesehen, wobei die Medikamenten-Wirkstofflösungen verschiedene Parameter und Inhaltsstoffe aufweisen und die Lösungsbestandteile verschiedene Parameter und Inhaltsstoffe aufweisen, und die Fördervorrichtung eine Mehrzahl an Zuführeinrichtungen und mindestens eine Speichereinrichtung zum Speichern der Parameter und/oder Inhaltsstoffe von mindestens zwei Medikamenten-Wirkstofflösungen und/oder Lösungsbestandteilen, welche durch die Mehrzahl an Zuführeinrichtungen zugeführt werden, aufweist. Dabei weist die Fördervorrichtung weiter eine mit der mindestens einen Speichereinrichtung verbundene Auswahleinrichtung zum Auswählen mindestens eines gespeicherten Parameters und/oder Inhaltsstoffes von jeder der mindestens zwei Medikamenten-Wirkstofflösungen und/oder Lösungsbestandteile durch einen Benutzer, eine mit der Auswahleinrichtung verbundene Berechnungseinrichtung zum Berechnen mindestens eines weiteren Parameters auf der Grundlage des mindestens einen ausgewählten Parameters und/oder Inhaltsstoffs, eine Steuereinrichtung zum Steuern des Betriebs mindestens einer weiteren Zuführeinrichtung zum Zuführen mindestens eines weiteren Medikamenten-Lösungsgemisches auf der Grundlage des berechneten weiteren Parameters, eine mit der Berechnungseinrichtung verbundene Blutzuckermesseinrichtung zum Messen eines Blutzuckerspiegels und eine mit der Berechnungseinrichtung verbundene Eingabeeinrichtung zum Eingeben körperspezifischer Daten wie Gewicht, Alter und Insulinsensivität eines Patienten auf, wobei das Medikamenten-Lösungsgemisch und das zumindest eine weitere Medikamenten-Lösungsgemisch ein Lösungsgemisch für die künstliche Ernährung sind, die Medikamenten-Wirkstofflösungen Nährstofflösungen sind, der Parameter der Nährstoffenergiegehalt der Nährstofflösung ist, der weitere Parameter eine Insulinrate darstellt und die Zuführeinrichtungen Infusionspumpen sind und für die Berechnung der Insulinrate die körperspezifischen Daten, die gemessenen Blutzuckerspiegelwerte des Patienten sowie die Nährstoffenergiegehälter und der zeitliche Verlauf der Zuführung der Nährstofflösungen durch die Mehrzahl an Zuführeinrichtungen verwendet werden.

Auf diese Weise wird ermöglicht, dass eine Mehrzahl an Wirkstoffen mit ihren dazugehörigen Parametern, wie beispielsweise die Dosis für die nachfolgende Berechnung der Förderraten oder die volumenunabhängigen Werkstoffmengen für die Aufstellung einer Bilanzierungsrechnung berücksichtigt werden, um hierdurch eine zutreffende und genaue Förderrate für das gesamte Medikamenten-Lösungsgemisch, was häufig bei der Behandlung von Patienten zur Erzielung eines Kombinationseffektes aufgrund der Verwendung mehrerer Medikamente erwünscht ist, oder eine exakte Bilanzierungsrechnung hinsichtlich der eingehenden und ausgehenden Volumina durch Berücksichtigung der Volumina der weiteren Medikamente mit weiteren Wirkstoffen oder hinsichtlich der Wirkstoffmengen, zu erhalten. Dies kann vorteilhaft dazuführen, dass zur Behandlung eines Patienten aufgrund der Berücksichtigung weiterer Medikamente andere ärztliche Entscheidungen getroffen werden, als wenn ausschließlich der Hauptwirkstoff bei den Berechnungen berücksichtigt worden wäre.

Erfindungsgemäß handelt es sich bei dem Medikamenten-Lösungsgemisch um ein Lösungsgemisch für die künstliche Ernährung mit verschiedenen Nährstoffen. Für die weiteren Lösungsbestandteile werden häufig Trägerlösungen als Trägermaterial für die Medikamentenwirkstoffe und Konservierungsstoffe zu Haltbarmachung der Lösungen verwendet. Bei den Inhaltsstoffen kann es sich um die in für Nährstofflösungen häufig verwendeten Nährstofftabellen aufgeführten Werte für Kohlenhydrate, Eiweiße und Fette handeln.

Bei einer derartigen künstlichen Ernährung wird eine automatisierte Verabreichung der Nährstofflösungen durchgeführt, wobei Informationen zu den Inhaltsstoffen und deren Eigenschaften bzw. Parametern umfassend, also für jede Nährstofflösung und nicht nur für die Nährstofflösung mit dem Hauptwirkstoff, in einer Speichereinrichtung bzw. einer Datenbank gespeichert werden.

Durch das Auswählen spezifischer Parameter wie dem Energiegehalt (in kJ) für die einzelnen Nährstofflösungen wird eine gegenseitige Berücksichtigung sämtlicher Nährstoffenergiegehälter bei einer Mehrzahl von verabreichten Nährstoffen für die Berechnung einer optimalen Förderrate und insbesondere für die Berechnung einer Insulinrate bei Diabetespatienten, die auf Intensivstation liegen, und deren Blutglukosespiegel auch von der Art der künstlichen Ernährung und damit von ihrem Energiegehalt abhängt, ermöglicht. Somit kann in Abhängigkeit von sämtlichen Nährstofflösungen und natürlich in Abhängigkeit von dem Blutglukosespiegel, der zu messen ist, und ggf. in Abhängigkeit weiterer personenspezifischer Daten, wie Gewicht, Alter und Insulinsensivität, anhand des Verlaufs der zugeführten Nährstoffenergiegehälter als Eingangsgrösse eine automatisierte Berechnung einer neuen Insulinrate durchgeführt werden, ohne dass hierdurch weitere Tätigkeiten eines Anwenders bzw. Bedienungspersonals der erfindungsgemäßen Fördervorrichtung notwendig sind.

Es wird somit sichergestellt, dass auch bei der Verwendung von Gemischen mit verschiedenen Medikamentenwirkstoffen eine sich aus der Summe der Wirkstoffe ergebende verbesserte Wirkung beim Patienten erhalten wird und nicht ausschliesslich der Hauptwirkstoff in seiner Wirkung betrachtet wird.

Vorteilhaft kann bei der Angabe der Nährstoffenergiegehälter als Parameter der Medikamenten-Wirkstofflösungen bzw. der Nährstofflösungen diejenige Energie mit berücksichtigt werden, die zusätzlich als Nebenwirkung bei der Infusion des Medikaments mit aufgenommen wird, wie beispielsweise bei Medikamenten, die in Sojamilch gelöst sind.

Die Berechnungseinrichtung ist zur Feststellung des Blutzuckerspiegels in dem Körper mit einer Blutzuckermesseinrichtung zum Messen des Blutzuckerspiegels und mit einer Eingabeeinrichtung zum Eingeben körperspezifischer Daten, wie dem Gewicht, dem Alter und Insulinsuffizienz verbunden.

Bei der künstlichen Ernährung kann eine parenterale, enterale oder eine Kombination beider vorliegen.

Das erfindungsgemäße Verfahren für die Zuführung des Medikamenten-Lösungsgemisches aus einer Mehrzahl an Medikamenten-Wirkstofflösungen und weiterer Lösungsbestandteile mittels einer Mehrzahl an Zuführeinrichtungen, wobei die Medikamenten-Wirkstofflösungen verschiedene Parameter und Inhaltsstoffe aufweisen und die Lösungsbestandteile verschiedene Parameter und Inhaltsstoffe aufweisen, weist vorteilhaft die Schritte des Speicherns der Parameter und/oder der Inhaltsstoffe von mindestens zwei Medikamenten-Wirkstofflösungen und/oder Lösungsbestandteilen in mindestens einer Speichereinrichtung, des Auswählens mindestens eines der gespeicherten Parameter und/oder Inhaltsstoffe von jeder der mindestens zwei Medikamenten-Wirkstofflösungen und/oder Lösungsbestandteilen mittels einer Auswahleinrichtung durch einen Benutzer, des Berechnens mindestens eines weiteren Parameters auf der Grundlage des mindestens einen ausgewählten Parameters und/oder Inhaltsstoffes von jeder der zumindest zwei Medikamenten-Wirkstofflösungen und/oder Lösungsbestandteilen und des Steuerns des Betriebs mindestens einer weiteren Zuführeinrichtung auf der Grundlage des berechneten weiteren Parameters auf, um mindestens ein weiteres Medikamenten-Lösungsgemisch, wie beispielsweise Insulin enthaltende Gemische, zuzuführen. Dabei weist das Verfahren weiter die Schritte des Messens eines Blutzuckerspiegels mittels einer mit der Berechnungseinrichtung verbundenen Blutzuckermesseinrichtung und des Eingebens körperspezifischer Daten wie Gewicht, Alter und Insulinsensivität eines Patienten mittels einer mit der Berechnungseinrichtung verbundenen Eingabeeinrichtung auf, wobei das Medikamenten-Lösungsgemisch und das zumindest eine weitere Medikamenten-Lösungsgemisch ein Lösungsgemisch für die künstliche Ernährung sind, die Medikamenten-Wirkstofflösungen Nährstofflösungen sind, der Parameter der Nährstoffenergiegehalt der Nährstofflösung ist, der weitere Parameter eine Insulinrate darstellt und die Zuführeinrichtungen Infusionspumpen sind und für die Berechnung der Insulinrate die körperspezifischen Daten, die gemessenen Blutzuckerspiegelwerte des Patienten sowie die Nährstoffenergiegehälter und der zeitliche Verlauf der Zuführung der Nährstofflösungen durch die Mehrzahl an Zuführeinrichtungen verwendet werden.

Bei der Auswahl der Nährstoffenergiegehälter als Parameter der Nährstofflösungen wird der zeitliche Verlauf der Zuführung der Nährstofflösungen durch eine Mehrzahl an Infusionspumpen beobachtet und für die Bestimmung einer neuen Insulinrate mit einbezogen.

Weitere vorteilhafte Ausführungsformen ergeben sich aus den Unteransprüchen.

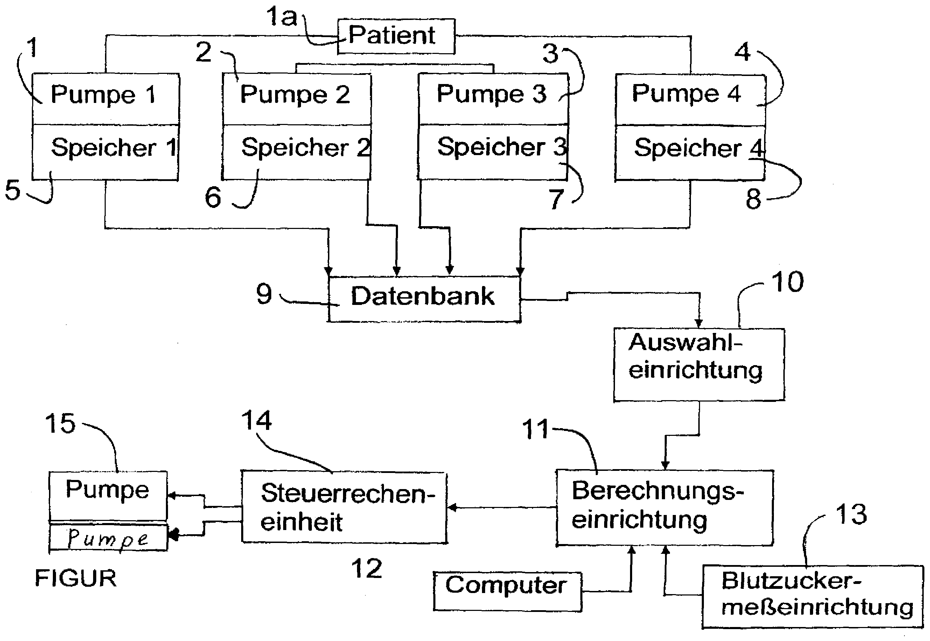

Vorteile und Zweckmäßigkeiten sind der nachfolgenden Beschreibung in Verbindung mit der Zeichnung zu entnehmen. Hierbei zeigt die Zeichnung gemäß der einzigen Figur eine schematische Darstellung der einzelnen Komponenten der erfindungsgemäßen Fördervorrichtung gemäß einer Ausführungsform der Erfindung.

In der Figur ist in schematischer Darstellung die erfindungsgemäße Fördervorrichtung wiedergegeben. Für eine enterale künstliche Ernährung eines Patienten 1a, der auf einer Intensivstation liegt, sind insgesamt vier Infusionspumpen 1-4 mit jeweils einer Speichereinheit 5-8 angeordnet, um den Patienten mit Nährstofflösungen, dazugehörigen Trägerlösungen und Konservierungsstofflösungen sowie ggf. weiteren Medikamentenlösungen zu versorgen.

In den Speichereinheiten 5-8 sind Daten zu den Nährstofflösungen und ggf. weiteren Medikamentenlösungen, die in den einzelnen Infusionspumpen 1-4 enthalten sind, abgelegt. Hierbei kann es sich beispielsweise um Parameter der Nährstofflösungen, wie deren Energiegehalt in Abhängigkeit von deren Inhaltsstoffen, nämlich Kohlenhydraten, Eiweißen und Fetten handeln. Selbstverständlich ist auch die Art der Nährstofflösung darin abgespeichert.

In einer gemeinsamen Datenbank 9 werden diese Daten abgespeichert, um von einem Bedienungspersonal ausgewählt werden zu können, wobei derselbe Parameter, beispielsweise der Energiegehalt, zu sämtlichen Lösungen ausgewählt wird.

In Abhängigkeit von dem Energiegehalt stellt ein Bedienungspersonal eine optimale Förderrate für die gesamte künstliche Ernährungslösung an jeder Pumpe ein.

In einer Auswahleinrichtung 10 findet die Auswahl des Parameters, in diesem Fall des Energiegehaltes, statt.

In einer Berechnungseinrichtung 11 wird in Abhängigkeit von dem zeitlichen Verlauf der dem Patienten zugeführten Energien in Abhängigkeit von sämtlichen Pumpen 1-4 ein weiterer Parameter, nämlich in diesem Fall einer neuen Insulinrate, berechnet.

Für die Berechnung der Insulinrate wird zusätzlich der aktuelle Blutglukosewert mittels einer Blutglukosemesseinrichtung 13 am Patienten gemessen und der Berechnungseinrichtung zugeführt.

Zudem werden mittels einer Eingabeeinrichtung 12 patientenspezifische Daten, wie das Gewicht, das Alter und die Insulinsensivität, beispielsweise mittels einer Tastatur eingegeben und der Berechnungseinrichtung für die Berechnung der neuen Insulinrate zugeführt.

Nachdem die Berechnungseinrichtung von der Berücksichtigung der eingegangenen oben aufgeführten Daten die neue Insulinrate automatisiert berechnet hat, wird diese als Ergebnis einer Steuerrecheneinheit 14 übertragen, welche daraufhin eine weitere Infusionspumpe 15, welche für das Zuführen von Insulin zu dem Patienten 1a zuständig ist, ansteuert und die Insulinzufuhr veranlasst.

Der Grundgedanke der erfindungsgemäßen Vorrichtung und des erfindungsgemäßen Verfahren ist auch auf das Zuführen eines jeden anderen Medikamentengemisches mit unterschiedlichen Wirkstoffen zu einem Patienten anwendbar.

Sämtliche in den Anmeldungsunterlagen offenbarten Merkmale werden als erfindungswesentlich beansprucht, sofern sie einzeln oder in Kombination gegenüber dem Stand der Technik neu sind.

1 : Pumpe 1
1a : Patient
2 : Pumpe 2
3 : Pumpe 3
4 : Pumpe 4
5 : Speicher 1
6 : Speicher 2
7 : Speicher 3
8 : Speicher 4
9 : Datenbank
10 : Auswahleinrichtung
11 : Berechnungseinrichtung
12 : Eingabeeinrichtung
13 : Blutzuckermesseinrichtung
14 : Steuerrecheneinheit
15 : Pumpe

**Patentansprüche**

1.    Fördervorrichtung für die Zuführung eines Medikamenten-Lösungsgemisches aus einer Mehrzahl an Medikamenten-Wirkstofflösungen und weiteren Lösungsbestandteilen, wobei
die Medikamenten-Wirkstofflösungen verschiedene Parameter und Inhaltsstoffe aufweisen und die Lösungsbestandteile verschiedene Parameter und Inhaltsstoffe aufweisen; und
die Fördervorrichtung aufweist:

eine Mehrzahl an Zuführeinrichtungen (1-4); und
mindestens eine Speichereinrichtung (9) zum Speichern der Parameter und/oder Inhaltsstoffe von mindestens zwei Medikamenten-Wirkstofflösungen und/oder Lösungsbestandteilen, welche durch die Mehrzahl an Zuführeinrichtungen (1-4) zugeführt werden, **dadurch gekennzeichnet, dass**
die Fördervorrichtung weiter aufweist:

eine mit der mindestens einen Speichereinrichtung (9) verbundene Auswahleinrichtung (10) zum Auswählen mindestens eines gespeicherten Parameters und/oder Inhaltsstoffes von jeder der mindestens zwei Medikamenten-Wirkstofflösungen und/oder Lösungsbestandteile durch einen Benutzer;

eine mit der Auswahleinrichtung (10) verbundene Berechnungseinrichtung (11) zum Berechnen mindestens eines weiteren Parameters auf der Grundlage des mindestens einen ausgewählten Parameters und/oder Inhaltsstoffs;

eine Steuereinrichtung (14) zum Steuern des Betriebs mindestens einer weiteren Zuführeinrichtung (15) zum Zuführen mindestens eines weiteren Medikamenten-Lösungsgemisches auf der Grundlage des berechneten weiteren Parameters;

eine mit der Berechnungseinrichtung (11) verbundene Blutzuckermesseinrichtung (12) zum Messen eines Blutzuckerspiegels; und

eine mit der Berechnungseinrichtung (11) verbundene Eingabeeinrichtung zum Eingeben körperspezifischer Daten wie Gewicht, Alter und Insulinsensivität eines Patienten,

wobei

das Medikamenten-Lösungsgemisch und das zumindest eine weitere Medikamenten-Lösungsgemisch ein Lösungsgemisch für die künstliche Ernährung sind, die Medikamenten-Wirkstofflösungen Nährstofflösungen sind, der Parameter der Nährstoffenergiegehalt der Nährstofflösung ist, der weitere Parameter eine Insulinrate darstellt und die Zuführeinrichtungen (1-4) Infusionspumpen sind; und

für die Berechnung der Insulinrate die körperspezifischen Daten, die gemessenen Blutzuckerspiegelwerte des Patienten sowie die Nährstoffenergiegehälter und der zeitliche Verlauf der Zuführung der Nährstofflösungen durch die Mehrzahl an Zuführeinrichtungen (1-4) verwendet werden.

**2.** Fördervorrichtung nach Anspruch 1, **dadurch gekennzeichnet, dass** die Lösungsbestandteile Trägerlösungen und Konservierungsstofflösungen sind.

**3.** Fördervorrichtung nach einem der vorangegangenen Ansprüche, **dadurch gekennzeichnet, dass** die Inhaltsstoffe Kohlenhydrate, Eiweiße und Fette sind.

**4.** Fördervorrichtung nach einem der vorangegangenen Ansprüche, **dadurch gekennzeichnet, dass** die künstliche Ernährung eine parenterale, enterale oder eine Kombination daraus ist.

**5.** Verfahren für die Zuführung eines Medikamenten-Lösungsgemisches aus einer Mehrzahl an Medikamenten-Wirkstofflösungen und weiteren Lösungsbestandteilen mittels einer Mehrzahl an Zuführeinrichtungen (1-4),

wobei die Medikamenten-Wirkstofflösungen verschiedene Parameter und Inhaltsstoffe aufweisen und die Lösungsbestandteile verschiedene Parameter und Inhaltsstoffe aufweisen; und

das Verfahren den Schritt aufweist:

Speichern der Parameter und/oder der Inhaltsstoffe von mindestens zwei Medikamenten-Wirkstofflösungen und/oder Lösungsbestandteilen in mindestens einer Speichereinrichtung (9), **dadurch gekennzeichnet, dass**

das Verfahren weiter die Schritte aufweist:

Auswählen mittels einer Auswahleinrichtung (10) mindestens eines der gespeicherten Parameter und/oder Inhaltsstoffe von jeder der mindestens zwei Medikamenten-Wirkstofflösungen und/oder Lösungsbestandteile durch einen Benutzer;

Berechnen mittels einer Berechnungseinrichtung (11) mindestens eines weiteren Parameters auf der Grundlage des mindestens einen ausgewählten Parameters und/oder Inhaltsstoffes von jeder der zumindest zwei Medikamenten-Wirkstofflösungen und/oder Lösungsbestandteilen;

Steuern des Betriebs mindestens einer weiteren Zuführeinrichtung (15) auf der Grundlage des berechneten weiteren Parameters, um mindestens ein weiteres Medikamenten-Lösungsgemisch zuzuführen;

Messen eines Blutzuckerspiegels mittels einer mit der Berechnungseinrichtung (11) verbundenen Blutzuckermesseinrichtung (12); und

Eingeben körperspezifischer Daten wie Gewicht, Alter und Insulinsensivität eines Patienten mittels einer mit der Berechnungseinrichtung (11) verbundenen Eingabeeinrichtung,

wobei

das Medikamenten-Lösungsgemisch und das zumindest eine weitere Medikamenten-Lösungsgemisch ein Lösungsgemisch für die künstliche Ernährung sind, die Medikamenten-Wirkstofflösungen Nährstofflösungen sind, der Parameter der Nährstoffenergiegehalt der Nährstofflösung ist, der weitere Parameter eine Insulinrate darstellt und die Zuführeinrichtungen (1-4) Infusionspumpen sind; und

für die Berechnung der Insulinrate die körperspezifischen Daten, die gemessenen Blutzuckerspiegelwerte des Patienten sowie die Nährstoffenergiegehälter und der zeitliche Verlauf der Zuführung der Nährstofflösungen durch die Mehrzahl an Zuführeinrichtungen (1-4) verwendet werden.

6. Verfahren nach Anspruch 5, **dadurch gekennzeichnet, dass** die künstliche Ernährung paraenteral, enteral oder mit einer Kombination daraus erfolgt.

7. Verfahren nach einem der Ansprüche 5 oder 6, **dadurch gekennzeichnet, dass** ein Warnhinweis bei Vorliegen einer Über- oder Unterdosierung des zugeführten Medikamenten-Lösungsgemisches gegeben wird.

## Claims

1. A conveyor apparatus for supplying a medicament solution mixture of a plurality of medicament active agent solutions and further solution constituents, wherein

   the medicament active agent solutions have different parameters and ingredients and the solution constituents have different parameters and ingredients; and

   said conveyor apparatus comprising:

   a plurality of feed devices (1-4); and
   at least one storage unit (9) for storing the parameters and/or the ingredients of at least two medicament active agent solutions and/or solution constituents being supplied by the plurality of feed devices (1-4), **characterized in that** the conveyor apparatus further comprises:

   a selection unit (10) connected to the at least one storage unit (9) for selecting at least one of the stored parameters and/or ingredients from each of the at least two medicament active agent solutions and/or solution constituents by a user;
   a calculation unit (11) connected to the selection unit (10) for calculating at least one additional parameter based on the at least one selected parameter and/or ingredient;
   a controller (14) for controlling the operation of at least one additional feed device (15) for supplying at least one additional medicament solution mixture based on the calculated additional parameter;
   a blood sugar measuring device (12) connected to the calculation unit (11) for measuring a blood sugar level; and
   an input device connected to the calculation unit (11) for inputting body-specific data such as weight, age and insulin sensitivity of a patient,
   wherein

the medicament solution mixture and the at least one additional medicament solution mixture are a solution mixture for artificial feeding, the medicament active agent solutions are nutrient solutions, the parameter is the nutrient energy content of the nutrient solution, the additional parameter is an insulin rate, and the feed devices (1-4) are infusion pumps; and

for the calculation of the insulin rate, the body-specific data, the measured blood sugar level values of the patient as well as the nutrient energy contents and the time curve of the supply of the nutrient solutions by the plurality of infusion pumps (1-4) are used.

2. The conveyor apparatus according to claim 1, **characterized in that** the solution constituents are carrier solutions and preservative solutions.

3. The conveyor apparatus according to one of the preceding claims, **characterized in that** the ingredients are carbohydrates, proteins and fats.

4. The conveyor apparatus according to one of the preceding claims, **characterized in that** the artificial feeding used is parenteral, enteral or a combination of the two.

5. A method for supplying a medicament solution mixture of a plurality of medicament active agent solutions and additional solution constituents by means of a plurality of feed units (1-4),
   wherein the medicament active agent solutions have various parameters and ingredients and the solution constituents have various parameters and ingredients, and
   the method comprises the step of:

   storing the parameters and/or the ingredients of at least two medicament active agent solutions and/or solution constituents in at least one storage unit (9), **characterized in that** the method additionally comprises the steps of:

   selecting by means of a selection unit (10) at least one of the stored parameters and/or ingredients from each of the at least two medicament active agent solutions and/or solution constituents by a user,
   calculating at least one additional parameter based on the at least one selected parameter and/or ingredient from each of the at least two medicament active agent solutions and/or the solution constituents by means of a calculation unit (11);
   controlling the operation of at least one additional feed unit (15) based on the calculated additional parameter, in order to supply at least one additional medicament solution mixture,

measuring a blood sugar level by means of a blood sugar measuring device (12) connected to the calculation unit (11); and inputting body-specific data such as weight, age and insulin sensitivity of a patient by means of an input device connected to the calculation unit (11),

wherein

the medicament solution mixture and the at least one additional medicament solution mixture are a solution mixture for artificial feeding, the medicament active agent solutions are nutrient solutions, the parameter is the nutrient energy content of the nutrient solution, the additional parameter is an insulin rate, and the feed devices (1-4) are infusion pumps; and

for the calculation of the insulin rate, the body-specific data, the measured blood sugar level values of the patient as well as the nutrient energy contents and the time curve of the supply of the nutrient solutions by the plurality of infusion pumps (1-4) are used.

6. The method according to claim 5, **characterized in that** the artificial feeding used is parenteral, enteral or a combination of the two.

7. The method according to one of claims 5 or 6, **characterized in that** a warning indication is given in the case of an over- or underdosing of the supplied medicament solution mixture.


## Revendications

1. Dispositif de transport pour l'amenée d'un mélange de solutions médicamenteuses composé d'une pluralité de solutions de principes actifs médicamenteuses et d'autres constituants de solution, dans lequel les solutions de principes actifs médicamenteuses présentent différents paramètres et ingrédients et les constituants de solution présentent différents paramètres et ingrédients ; et

le dispositif de transport présente :

une pluralité de systèmes d'amenée (1-4) ; et au moins un système de mémoire (9) servant à mémoriser les paramètres et/ou ingrédients d'au moins deux solutions de principes actifs médicamenteuses et/ou de composants de solution, lesquels sont amenés par la pluralité de systèmes d'amenée (1-4), **caractérisé en ce que**

le dispositif de transport présente en outre :

un système de sélection (10) relié à l'au moins un système de mémoire (9), servant à sélectionner au moins un paramètre et/ou ingrédient mémorisé de chacune/chacun des au moins deux solutions de principes actifs médicamenteuses et/ou composants de solution par un utilisateur :

un système de calcul (11) relié au système de sélection (10), servant à calculer au moins un autre paramètre sur la base de l'au moins un paramètre et/ou ingrédient sélectionné ;

un système de commande (14) servant à commander le fonctionnement d'au moins un autre système d'amenée (15) servant à amener au moins un autre mélange de solutions médicamenteuses sur la base de l'autre paramètre calculé ;

un système de mesure de glycémie (12) relié au système de calcul (11), servant à mesurer un niveau de glycémie ; et

un système de saisie relié au système de calcul (11), servant à saisir des données spécifiques au corps telles que le poids, l'âge et l'insulinosensibilité, d'un patient,

dans lequel

le mélange de solutions médicamenteuses et l'au moins un autre mélange de solutions médicamenteuses sont un mélange de solutions pour la nutrition artificielle, les solutions de principes actifs médicamenteuses sont des solutions nutritives, le paramètre est la teneur en énergie nutritive de la solution nutritive, l'autre paramètre constitue un taux d'insuline et les systèmes d'amenée (1-4) sont des pompes de perfusion ; et les données spécifiques au corps, les valeurs de niveau de glycémie mesurées du patient ainsi que les teneurs en énergie nutritive et l'évolution dans le temps de l'amenée des solutions nutritives par la pluralité de systèmes d'amenée (1-4) sont utilisées pour le calcul du taux d'insuline.

2. Dispositif de transport selon la revendication 1, **caractérisé en ce que** les constituants de solution sont des solutions porteuses et des solutions de conservateurs.

3. Dispositif de transport selon l'une quelconque des revendications précédentes, **caractérisé en ce que** les ingrédients sont des glucides, des protéines et des matières grasses.

4. Dispositif de transport selon l'une quelconque des revendications précédentes, **caractérisé en ce que** la nutrition artificielle est une nutrition parentérale, entérale ou une combinaison de celles-ci.

5. Procédé pour l'amenée d'un mélange de solutions médicamenteuses composé d'une pluralité de solutions de principes actifs médicamenteuses et d'autres composants de solution au moyen d'une pluralité de systèmes d'amenée (1-4),
dans lequel les solutions de principes actifs médicamenteuses présentent différents paramètres et ingrédients et les composants de solution présentent différents paramètres et ingrédients ; et
le procédé présente l'étape :

de mémorisation des paramètres et/ou des ingrédients d'au moins deux solutions de principes actifs médicamenteuses et/ou de composants de solution dans au moins un système de mémorisation (9), **caractérisé en ce que**
le procédé présente en outre les étapes :

de sélection au moyen d'un système de sélection (10) d'au moins un des paramètres et/ou ingrédients mémorisés de chacune/chacun des au moins deux solutions de principes actifs médicamenteuses et/ou composants de solution par un utilisateur ;
de calcul au moyen d'un système de calcul (11) d'au moins un autre paramètre sur la base de l'au moins un paramètre et/ou ingrédient sélectionné de chacune/chacun des au moins deux solutions de principes actifs médicamenteuses et/ou composants de solution ;
de commande du fonctionnement au moins d'un autre système d'amenée (15) sur la base de l'autre paramètre calculé pour amener au moins un autre mélange de solutions médicamenteuses ;
de mesure d'un niveau de glycémie au moyen d'un système de mesure de glycémie (12) relié au système de calcul (11) ; et
de saisie de données spécifiques au corps telles que le poids, l'âge et l'insulinosensibilité d'un patient au moyen d'un système de saisie relié au système de calcul (11), dans lequel
le mélange de solutions médicamenteuses et l'au moins un autre mélange de solutions médicamenteuses sont un mélange de solutions pour la nutrition artificielle, les solutions de principes actifs médicamenteuses sont des solutions nutritives, le paramètre est la teneur en énergie nutritive de la solution nutritive, l'autre paramètre constitue un taux d'insuline et les systèmes d'amenée (1-4) sont des pompes de perfusion ; et
les données spécifiques au corps, les valeurs de niveau de glycémie mesurées du patient ainsi que les teneurs en énergie nutritive et l'évolution dans le temps de l'amenée des solutions nutritives par la pluralité de systèmes d'amenée (1-4) sont utilisées pour le calcul du taux d'insuline.

6. Procédé selon la revendication 5, **caractérisé en ce que** la nutrition artificielle est effectuée de manière parentérale, entérale ou avec une combinaison de celles-ci.

7. Procédé selon l'une quelconque des revendications 5 ou 6, **caractérisé en ce qu'**une mise en garde est fournie en présence d'un surdosage ou d'un sous-dosage du mélange de solutions médicamenteuses amené.

FIGUR

EP 2 195 758 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20030032867 A1
- US 6827702 B2
- WO 2004069095 A

**In der Beschreibung aufgeführte Nicht-Patentliteratur**

- **MADSEN, H. et al.** The Hitchhiker's Guide to Parenteral Nutrition Management for Adult Patients. *Practical Gastroenterology,* Juli 2006
- **SACKS, G. S.** Drug-Nutrient Considerations in Patients Receiving Parenteral and Enteral Nutrition. *Practical Gastroenterology,* Juli 2004